(19) Europäisches Patentamt
European Patent Office
Office européen des brevets

(11) Publication number: 0 236 007 B1

(12) # EUROPEAN PATENT SPECIFICATION

(45) Date of publication of the patent specification: 29.08.90

(51) Int. Cl.[5]: **C07C 59/305**, C07C 51/347

(21) Application number: 87301376.7

(22) Date of filing: 18.02.87

(54) **Process for the preparation of oxodisuccinates useful as detergent builders.**

(30) Priority: 24.02.86 US 834185
04.02.87 US 5633

(43) Date of publication of application: 09.09.87 Bulletin 87/37

(45) Publication of the grant of the patent: 29.08.90 Bulletin 90/35

(84) Designated Contracting States: BE DE FR GB IT LU NL

(56) References cited:
US-A- 3 128 287
US-A- 3 635 830
US-A- 4 017 541

(73) Proprietor: THE PROCTER & GAMBLE COMPANY, One Procter & Gamble Plaza, Cincinnati Ohio 45202(US)

(72) Inventor: Bush, Rodney Dean, 1778 Clayburn Circle, Cincinnati Ohio 45240(US)
Inventor: Heinzman, Stephen Wayne, 515 West McMillan Avenue, Cincinnati Ohio 45219(US)
Inventor: Connor, Daniel Stedman, 9217 Sagemeadow Drive, Cincinnati Ohio 45239(US)
Inventor: Kretschmar, Herbert Charles, 160 Julep Lane, Cincinnati Ohio 45218(US)
Inventor: Mackey, Larry Neil, 5856 Crestview Avenue, Fairfield Ohio 45014(US)

(74) Representative: Brooks, Maxim Courtney et al, Procter & Gamble (NTC) Limited Whitley Road Longbenton, Newcastle-upon-Tyne NE12 9TS(GB)

EP 0 236 007 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an improved process for making certain ether carboxylate salts, particularly preferred being 2,2'-oxodisuccinate salts. Sodium forms thereof are particularly useful, safe, biodegradable ether carboxylate sequestrants or builders in detergent compositions for household, institutional and industrial use.

A number of types of ether polycarboxylates are known in the art and, along with methods for their preparation, have been disclosed in the patent literature. For example, Stubbs et al, US-A 4 017 541, issued April 12, 1977, disclose carboxyalkyl ethers said to include alkali metal salts of propylene glycol monosuccinyl ether and propylene glycol disuccinyl ether. Pearson et al, US-A 3 776 850, issued December 4, 1973, disclose polycarboxylates said to have a formula which corresponds to HO–[C(R)-(COOH)CR(COOH)O]$_n$–H wherein R can be hydrogen and n ranges from 2 to 4.

Berg, US-A 3 128 287, issued April 7, 1964, and Lamberti et al, US-A 3 635 830, issued January 18, 1972 both disclose 2,2'-oxodisuccinic acid and salts thereof, which are prepared by heating maleic anhydride or maleic anhydride or maleic acid in the presence of a molar excess of calcium hydroxide, followed by acid treatment of the resulting reaction product. The '830 patent discloses the use of this oxodisuccinic acid material as a detergent builder. Bush, US-A 4 566 984, issued January 28, 1986, discloses other polycarboxylate builders.

The disclosed methods for preparing the ether polycarboxylates of the foregoing patents in general involve 1) combining acidic organic reactants and an alkaline earth metal base; 2) carrying out an ether-bond forming reaction wherein the organic reactants and an alkaline earth metal base; 2) carrying out an ether-bond forming reaction wherein the organic reactants couple in the presence of excess alkaline earth metal by a Michael-type addition, and 3) isolation or purification to remove the alkaline earth metal or organic by-products. In the above, the ether-bond forming step 2) is particularly characterized in invariably using a single metal, which is an alkaline earth, present in stoichiometric or superstoichiometric amounts; further, in that a two-phase solid-liquid reaction is involved. Even relatively recent disclosures of ether-bond forming reactions do not focus on this step, which may, according to the art, involve no more than crudely combining organic reactants with base in the absence of solvent. Known ether-forming reactions of particular ether carboxylates have slow reaction kinetics with respect to the desired products. Side reactions, e.g., formation of fumarate may occur in parallel to a greater extent than would be the case were the ether-formation reaction to occur rapidly. Ether carboxylate isolation steps subsequene to known ether-forming reaction steps frequently involve difficult, cumbersome and uneconomical purification; the finely-divided solid alkaline earth salts and the organic by-products must be removed from the product. These processing disadvantages have proved surprisingly intractable over the years and render particular ether carboxylate materials such as 2,2'-oxodisuccinate salts unattractive for use as builders in detergent products to be commercially marketed in large volume, despite their biodegradability and efficacy.

The search for improved ether carboxylate purification processes is illustrated by the disclosures of Blumbergs et al, US-A 3 821 296, issued June 28, 1974; Lindsay et al, US-A 3 862 219, issued January 21, 1975; and Blumbergs et al US-A 3 948 985, issued April 6, 1976. The '296 patent discloses a crystallization enhancement process for converting to the trisodium salt a crude calcium salt of CMOS (in the form of a mixture obtained by reacting maleic and glycolic acids in the presence of $Ca(OH)_2$). In the disclosed process, soda ash and the abovementioned mixture are added in about stoichiometric amounts to a preformed "heel". Said heel comprises product of the prior combination of soda ash with some of the abovementioned mixture. The '219 patent discloses another purification process; zinc or alkaline earth metal carboxyalkoxysuccinates, preformed from maleic acid and a hydroxyorganic acid, are converted into sodium salt form by treatment with substoichiometric amounts of carbonate. The product derived by mixing these components comprises a solution of sodium carboxyalkoxysuccinate salts, and solide alkaline earth metal salts. The solution is separated and evaporated to obtain sodium salts in solid form. The '985 patent discloses preparation of the acid form of CMOS by acidifying the calcium salt of CMOS (prepared from maleic anhydride and glycolic acid with excess ($Ca(OH)_2$) with sulfuric acid and filtration of $CaSO_4 \cdot 2H_2O$. The latter readily precipitates at elevated temperatures between about 40 and 80°C. Fumarate by-product is removed from the carboxymethyl oxysuccinic acid by conventional methods of extraction with organic solvents. Irrespective of these developments, all of which focus on purification of preformed ether carboxylates, there has been no recognition that simple modification of the ether formation step might lead more directly to a truly viable commercial process for ether carboxylate synthesis. Accordingly, it is an object of the present invention to provide an improved 2,2'-oxodisuccinate synthesis method wherein the ether-bond forming process is carried out efficiently, conveniently and rapidly. Contrasting with the art, homogeneous and, in certain cases, clear liquid reaction mixtures can be formed. These are readily pumpable or otherwise workable at high concentrations of reactants, and provide 2,2'-oxodisuccinate calcium and single cation-salts readily isolable or convertible to sodium salt form by conventional process steps.

The present invention relates to an improved process for forming sodium and calcium ether carboxylates. A reactant component comprising organic ether-bond forming acids and sodium or calcium salts or mixtures thereof are reacted in a homogeneous alkaline environment wherein both sodium and calcium

are also present in particular amounts. The calcium content of the reaction mixture is sub-stoichiometric with respect to the moles of organic reactant component which are used.

The process of the invention is particularly suitable for forming sodium and calcium 2,2'-oxodisuccinates which are readily convertible into a form suitable for use as detergency builders by means of conventional, art disclosed processes. Said 2,2'-oxodisuccinate salts are prepared according to the process of the invention by a process which involves combining particular inorganic and organic reactant components at temperatures below 120°C to form an alkaline aqueous ether-bond forming reaction mixture, which has, as initially formed, a specific range of composition. This mixture is maintained at a temperature of at least 60°C sufficient to permit ether-bond formation to occur. In the initially formed reaction mixture is present an ether-bond forming combination of maleate and malate. The maleate is derived from maleate reactants which are acids or salts of maleate (empirical formula $C_4H_2O_4$) with acidic hydrogen, sodium, calcium or combinations thereof. The malate is derived from malate reactants which are acids or salts of malate (empirical formula $C_4H_4O_5$) with acidic hydrogen, sodium, calcium or combinations thereof. Likewise present is at least one inorganic base or combination of said base with an inorganic salt, the amount and compositions of which are such that the initially formed reaction mixture has a maleate/malate molar ratio ranging from 1:1 to 2:1, a calcium to maleate plus malate molar ratio ranging from 0.1:1 to 0.75:1 and a sodium to maleate plus malate molar ratio ranging from 0.5:1 to 2.2:1. The amount of inorganic based added in forming the reaction mixture is preferably sufficient both to completely neutralize said mixture and to yield a molar excess of hydroxide ranging from 0.01 to 0.4 moles of hydroxide per mole of combined maleate and malate. Maleate plus malate are generally present in the reaction mixtures, as initially formed, in concentrations (calculated as $C_4H_2O_4$ and $C_4H_4O_5$, respectively) ranging from 31% to 41% by weight of the total mixture. Reaction mixtures of the invention are particularly useful in being homogeneous low viscosity fluids forming rapidly, conveniently and economically the sodium and calcium 2,2'-oxodisuccinate salts in good yield.

Particularly preferred processes of the invention use maleic acid or its anhydride and malic acid as the organic reactant component and sodium and calcium hydroxides together as the inorganic reactant component. Preferred reactant combination and ether-bond forming reaction temperatures range from 65 to 85°C, and at the most highly preferred of such temperatures, which is about 75°C, excellent yields of sodium and calcium 2,2'-oxodisuccinate are obtained in periods of from about 1 to about 6 hours. Continuous processes could be readily provided on this basis for high conversions of the maleate and malate reactants into 2,2'-oxodisuccinate salts.

Also included herein are highly preferred processes for forming the 2,2'-oxodisuccinate salts by combining in particular sequences through intermediate reactant combinations, the above-named preferred reactants; the interrelated process conditions to carry out these combinations and the subsequent ether-bond forming reactions most advantageously, are given in full.

## DETAILED DESCRIPTION OF THE INVENTION

### Reactants

#### Organic Reactant Component

The processes herein comprise combining an organic reactant component (which may itself include metal cations) and an inorganic reactant component, then conducting an ether-bond-forming reaction. Careful specification of the forms, composition and proportions of the reactants is required. For example, the organic reactant component may change chemical form by hydration, acid-base reaction or cation exchange, which are rapid reactions capable of occurring prior to any of the ether-bond forming reactions of primary interest herein.

All the organic reactants herein comprise maleate reactants and malate reactants. These are organic carboxylate compositions wherein essentially no ether (C-O-C) bonds are present, but which are capable of releasing maleate or malate species which form ether bonds under the process conditions described herein. Irrespective of the form of such reactants, physical or chemical, prior to use in the process of the invention, they will be capable of yielding maleate (empirical formula $C_4H_2O_4$) or malate (empirical formula $C_4H_4O_5$) in particular molar amounts at some "initial" time after they are combined in an ether-bond forming reaction mixture of the invention. The "initial" time is defined to be that time after combining organic and inorganic reactants at which no ether-bond forming or organic chemical reaction (other than maleic anhydride conversion to acid form) has yet occurred, so that the molar amounts of maleate or malate present are given directly by, and may be calculated from the weights and compositions of the maleate and malate reactants used. The initial molar amounts of maleate and malate can then be used as an unambiguous basis for specifying the relative molar proportions of other reactant species, e.g., sodium, calcium, alkali, which require also to be present in the ether-bond forming reaction mixtures herein.

It will also be understood that irrespective of the true degree of dissociation in the concentrated aqueous media of the processes herein, which may suppress such ion dissociation, empirical formula units of

maleate and malate herein have structures consistent with (I) and (II), respectively, and would be expected to take the fully dissociated anionic forms depicted, in dilute aqueous solution.

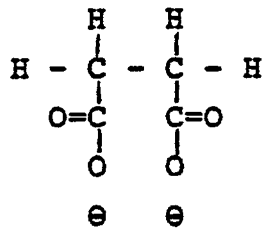
(I)

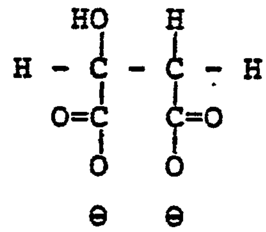
(II)

It should be recognized that malate can take different stereochemical forms, such as DL-malate, herein.

The chemically suitable forms of maleate and malate reactants herein need only comprise acids (the anhydride of maleic acid being included), simple water-soluble salts of single cations, especially sodium or calcium, or salts believed to have more complex structures, releasably containing maleate, malate, acidic hydrogen, sodium, calcium or combinations thereof in particular molar amounts based upon given weights of the reactants taken, combinations and hydrated forms of said reactants being included.

Illustrative of suitable maleate reactants are maleic anhydride, maleic acid, sodium maleate and calcium maleate. Calcium maleate may be prepared by reacting maleic acid or maleic anhydride with calcium carbonate. Illustrative of suitable malate reactants are malic acid, sodium malate and calcium malate prepared by art-disclosed processes.

Illustrative of the complex salt compositions included in defining the organic component are a maleate composition of empirical formula $[NaH(C_4H_2O_4)]$ and a malate composition of empirical formula $[Ca(Na(C_4H_4O_5))_2]$ which are acidic- or partial-salt, and dianionic compositions, respectively. Polyanionic compositions are likewise included.

Maleate and malate reactant compositions herein are not particularly limited with respect to the inclusion of materials chemically inert under the reaction conditions herein, or limited with respect to physical form, provided that based upon given weights, said reactant compositions yield particular molar amounts of maleate or malate. Solids, liquids or solutions are acceptable. For practical purposes, however, it is generally desirable to use maleate and malate reactants of commercial quality. Such reactants will often contain impurities, for example, in the form of fumarates. Maleate reactants may contain malate, usually in minor amounts. (It will be appreciated that maleate can isomerize to fumarate, at least to some extent, or undergo conversion to malate as is known in the art, thus affecting to some extent the precise compositions of the organic reactant component.)

Based on the above definition, it will be understood what is intended in stating that the organic reactant component used in the processes of the present invention consists essentially of

a) at least one maleate reactant selected from
i) acidic or fully neutralized salts, formed from maleate having the empirical formula $C_4H_2O_4$, and acidic or neutralizing moieties selected from H, sodium and calcium, and combinations thereof,
ii) maleic anhydride,
iii) maleic acid, and
iv) mixtures of said maleate reactants; and
b) at least one malate reactant, in the form of a single isomer or of isomeric mixtures, selected from
i) acidic or fully neutralized salts, formed from malate having the empirical formula $C_4H_4O_5$, and acidic or neutralizing moieties selected from H, sodium and calcium, and combinations thereof,
ii) malic acid, and
iii) mixtures of said malate reactants.

Notwithstanding the above definition of maleate reactants used herein, the ether-bond forming process of the present invention is believed applicable to water-soluble reactants derived from a wide variety of acids and salts (of types defined above) yielding in aqueous solution the conjugated structure (III) wherein X and Y are any groups compatible with ether-bond forming reactions, as defined herein, which are conducted in basic aqueous media.

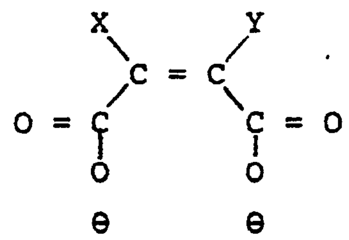

(III)

Likewise, notwithstanding the above definition of malate reactants used herein, the ether-bond forming process of the present invention is believed applicable to water-soluble reactants derived from a wide variety of moieties having structure IV or V wherein Z, R′, $R^2$ and $R^3$ are any group compatible with ether-bond forming reactions conducted in aqueous media, provided that gem-dihydroxy and hemiacetal derivatives are specifically excluded.

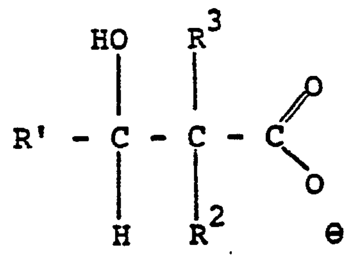

(IV)

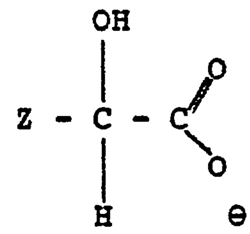

(V)

Inorganic Reactant Component

The ether-bond formation reactions herein require, in addition to the above-defined organic reactant component, a particular inorganic reactant component, the composition of which should be suitable to form, when combined with said organic reactant component, an alkaline reaction mixture comprising both sodium and calcium in particular amounts as further defined hereinafter.

The inorganic reactant component will in general consist essentially of at least one inorganic base capable of yielding hydroxide anions, at least in dilute aqueous solution. The inorganic base will commonly be selected from the oxides and hydroxides of sodium, calcium or mixtures thereof, though subject to the sodium, calcium and alkalinity constraints upon the ether-bond forming reactions herein, other bases could conceivably be used.

The inorganic reactant component can alternatively comprise mixtures of said inorganic base with water-soluble inorganic salts capable of yielding sodium, calcium or mixtures thereof together with anions inert for the purposes of the ether bond-forming reactions herein. These salts function exclusively to provide an alternative means of conveying particular amounts of sodium or calcium into the ether-bond forming reaction mixture. As such, their anion composition is not of particular importance, provided that the anions do not interact detrimentally with the ether-bond forming reaction mixture. Suitable salts, for example, include sodium sulfate, sodium bisulfate, sodium chloride and calcium chloride. In contrast, an unsuitable salt is calcium carbonate. Being insoluble, this salt cannot be used in the present process as the inorganic reactant component without modifying said process. Further examples of unsuitable salts are sodium peroxides and hypochlorites in which the anions are capable of chemically reacting with the maleate and malate in the ether-bond forming reaction mixtures. Although it has not been demonstrated that other sodium or calcium salts of inert anions are equally effective or cost-effective, it is conceivable that alternative suitable inorganic salts could be used in the processes herein.

The inorganic component herein is not necessarily limited as to physical form. Hydrates, slurries, and solutions or dry materials can be used subject to the composition requirements of ether-bond forming reactions further defined hereinafter. It will generally be convenient to use commercial grades of inorganic reactants.

Recognizing that the organic reactant component defined above may provide a source of acidity, of sodium, calcium or all three in the processes herein, and also that said inorganic component may comprise inorganic salts, it is convenient herein to calculate the required total molar composition of sodium, calcium and alkali in the ether-bond forming reaction mixtures based upon the moles of maleate plus malate to be used. By means of the strict sodium, calcium and alkali (i.e., base) molar ratios to maleate and malate required in the ether-bond forming reactions given hereinafter, the practitioner of this invention will be able simply to calculate the molar amounts of the inorganic reactant component needed for reacting any selected amount of maleate and malate according to the process of the invention.

It will be appreciated that the processes herein are desirably conducted at high concentrations in aqueous media as further defined hereinafter, both due to their efficacy and high throughput, permitting more economical plant. Preferred reactant compounds, at least in part based upon this observation, are together maleic acid or maleic anhydride, malic acid (particularly preferred is DL-malic acid) and sodium and calcium hydroxides. Maleic acid or maleic anhydride, and malic acid together deliver high maleate and malate molar amounts per unit weight. The combination of sodium and calcium hydroxides is capable of delivering alkalinity in the form of hydroxide and the particular sodium and calcium composition ranges shown unexpectedly herein to be essential to improved ether-bond formation processes.

The Ether-Bond Forming Reaction

It has been discovered that the use of combinations of sodium and calcium, rather than purely calcium, in aqueous alkaline ether-bond formation reactions, leads unexpectedly to rapid, high yield ether carboxylate production in ultimately homogeneous high-concentration reaction mixtures wherein less than one mole of calcium is present for every mole of maleate and malate used.

It is believed that the improved ether-bond formation may be broadly applicable as indicated above in defining maleate and malate reactants and moieties. For example, processes of the present invention could be used to prepare the novel ether carboxylates comprising mixtures of tartrate monosuccinates and tartrate disuccinates by this process disclosed in EP-A 232 114.

The following method is particularly applicable to the production of sodium and calcium 2,2′-oxodisuccinates. These ether carboxylate salt compositions consist essentially of particular amounts of 2,2′-oxodisuccinate, having empirical formula ($C_8H_6O_9$), together with particular amounts of sodium and calcium. The structure of the 2,2′-oxodisuccinate tetra-anion is illustrated below.

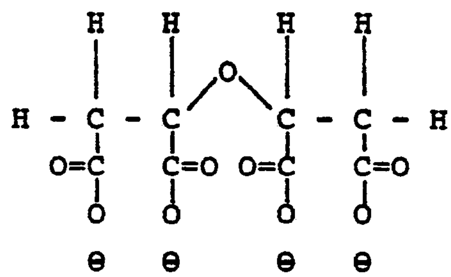

(VI) = ODS

The overall sodium, calcium and minor by-product composition of the 2,2′-oxodisuccinate compositions formed will to some extent be dependent upon which particular embodiment of the process described below is used.

The process for forming sodium and calcium 2,2′-oxodisuccinates comprises (I) combining, at temperatures below 120°C, in aqueous media to form a reaction mixture, an organic reactant component, consisting essentially of a) at least one maleate reactant and b) at least one malate reactant, together with an inorganic reactant component, consisting essentially of a) at least one inorganic base or b) of mixtures of said base with particular inorganic salts, all reactants as hereinbefore defined and in specific proportions given below; and (II) maintaining said reaction mixture at a temperature of at least 60°C, sufficient to permit ether-bond formation between said maleate and malate present in the reaction mixture. In the reaction mixture of the invention, maleate:malate molar ratios should range from 1:1 to 2:1, more preferably 1.1:1 to 1.6:1, at the "initial" time of combination. Maleate and malate amounts will, of course, decrease, and proportions may vary during the course of the 2,2′-oxodisuccinate ether-bond forming reaction since some degree of maleate → fumarate and maleate → malate conversion will concurrently occur.

It is furthermore essential that in forming said reaction mixture, the "initial" molar ratio of calcium to maleate plus malate should be from 0.1:1 to 0.75:1, more preferably from 0.31:1 to 0.57:1. Likewise it is essential that in forming said reaction mixture, sodium should be present in an "initial" molar ratio of sodium to maleate plus malate of from 0.5:1 to 2.2:1. When maleate and malate reactants are in acid form and no inorganic salts are used, the sodium to maleate plus malate molar ratio should preferably be within a range of from 0.9:1 to 1.48:1. Finally, sufficient inorganic base should be added to the reaction mixture to render said mixture alkaline. Preferably, inorganic base is added in an amount to fully neutralize said reaction mixture and in an amount further sufficient to yield an excess of from 0.01 to 0.4, more preferably from 0.04:1 to 0.1:1 moles of hydroxide per mole of combined maleate plus malate. Specifying the alkalinity in this manner has proved consistently more reliable than pH measurement; nonetheless by sampling the reaction mixture, cooling the sample to 25°C and measuring pH, pH values obtained should lie from 9 to 13; pH values in the upper half of this range are generally preferred.

It is also preferable to conduct the ether-bond forming reaction within a high, narrowly defined range of concentrations, by combining the inorganic and organic reactant components in aqueous media to provide total initial concentrations of maleate plus malate, of from 31% to 41%, more preferably from 36% to 40%.

Desirably, all organic and inorganic reactants will be combined using physical agitation (with the clear purpose in mind of achieving good mixing as well as of conducting the combination and subsequent reaction safely in view of the exothermic nature of certain of the hydration or neutralization reactions which occur). Temperatures of combination of the reactants should be constrained with a range of from 60°C to 120°C, though lower temperatures may be permissible in limited circumstances; e.g., when the organic reactants are in salt form. It is not generally advisable to permit combination temperatures below 60°C owing to the tendency of certain salts, e.g., [$HNa(C_4H_2O_4)$], to precipitate. Temperatures above 120°C may increase formation of by-products and at temperatures above the boil it is preferable to provide some means of condensing water which evaporates, so that it returns to the reaction mixture. The reaction temperatures defined above are generally maintained for a period of from 0.5 to 12 hours.

Highly preferred reactant combination and ether-bond forming reaction temperatures range from 65° to 85°C. Reaction temperature and time herein are apparently correlated such that preferred combinations involve reaction times of from 1 to 6 hours at the most highly preferred reaction temperature, which is about 75°C. Preferred reaction temperatures and times herein are based on optimization of yield and product purity.

By sampling the reaction mixtures at intervals during ether-bond formation, and applying techniques such as HPLC chromatography, the yield of 2,2′-oxodisuccinates and the levels of maleate and malate reactants and of fumarate by-products in any individual reaction can be monitored. It is believed that yields of 2,2′-oxodisuccinates obtained according to the ether-bond-forming process herein may reach a maximum over a period of time and then decrease.

Ether-bond forming reactions herein should be arrested by cooling, typically to below 50°C, and preferably to ambient temperature, at such time as when yields of at least 50% by weight of the organic species present (typically, maleate plus malate plus fumarate plus 2,2′-oxodisuccinate) are in 2,2′-oxodisuccinate converted form.

The reactions herein proceed at low viscosity, and the ether-formation reaction mixtures herein are readily stirred, pumped, etc. and form ultimately a single phase, which is a clear liquid. Even at the outset of ether-bond forming reactions herein, the reaction mixtures (upon formation with careful mixing) are homogeneous, easily pumpable fluids. This contrasts with the art (see, for example, US-A 3 635 830) wherein large amounts of insoluble salts of the reactants are formed. It is believed that in situ generated complex sodium/calcium salts, of the maleate and malate reactants, as well as of the 2,2′-oxodisuccinate products, have much higher solubilities than the single-metal calcium salts, thereby allowing for the convenient high-concentration processes herein. The kinetics of 2,2′-oxodisuccinate formation are also significantly enhanced by the co-presence of all the reactants, including calcium, ultimately in a single phase, thereby providing a workable and efficient ether carboxylate production process.

The ether-bond formation reactions herein can generally be carried out in a stainless steel apparatus equipped with a source of agitation, e.g., a mechanical stirring device, and with cooling means, e.g., a cooling surface such as a jacket. Warm, rather than cold, water is used as a coolant and the preferred reaction apparatus will be designed to maximize shear over the surfaces of the cooling surface to ensure that encrustation by reactants or products does not occur, or is minimized.

In defining the ether-bond forming process of the invention, it is intended to include both batch and continuous processes which result in ether-bond forming reaction mixtures having composition ranges given herein, optionally through the use of reactant or product recycle steps.

## Preferred Ether-Bond Forming Reactions

Two processes of the present invention are illustrative of the preferred reactants, conditions and composition ranges in the practice of this invention. These processes are also illustrative of certain intermediate compositions which can be prepared by combining reactants herein.

The intermediate composition ranges involved in these processes are selected to result consistently in ether-bond forming reaction mixtures having the particular composition requirements of the invention.

In one such process for forming 2,2′-oxodisuccinate salts, the following sequence is used:

i) Dry solid calcium hydroxide is added to an aqueous sodium hydroxide solution, to form an alkaline slurry comprising from 16 to 20% by weight $Ca(OH)_2$ and correspondingly from 30 to 34% by weight NaOH; so that the Ca:Na mole ratio therein is from 0.2 :1 to 0.4:1.

ii) Maleic anhydride or maleic acid are combined together, and DL-malic acid, using a maleate/malate mole ratio of from 1.4:1 to 1.6:1, is added at a temperature from 65 to 85°C, to form an acidic aqueous solution wherein the concentration of maleate plus malate is from 65 to 75%.

iii) The alkaline slurry of step i) and the acidic solution of step ii) are combined with agitation, at a rate selected to minimize precipitation of partially neutralized salts of maleic acid and to form a homogeneous mixture, at a temperature of from 65 to 85°C, such that the mole ratio of calcium to maleate plus malate

combined is from 0.35:1 to 0.45:1 and the mole ratio of sodium to maleate plus malate combined is from 1.25:1 to 1.35:1.

iv) The reaction mixture which is formed is mixed and maintained at a temperature from 65°C to 85°C, until at least 50% by weight of the organic species present are in the form of 2,2′-oxodisuccinates. HPLC and atomic absorption analysis can be used to check organic composition and sodium/calcium content, respectively.

v) The reaction mixture is cooled to arrest chemical reaction. Typically, cooling to less than 50°C, more preferably to ambient temperature, is carried out.

vi) The calcium content of the reaction mixture is reduced using conventional methods of precipitation or sequestration so that calcium comprises from 0.2 to 1% by weight of 2,2′-oxodisuccinate salts present. Ethane hydroxy diphosphonate, an acidic resin, sodium carbonate, sodium bicarbonate, or combinations thereof are used for this purpose.

An alternative process for forming 2,2′-oxodisuccinate salts, uses the following sequence:

i) An alkaline slurry is prepared by combining calcium hydroxide and aqueous sodium hydroxide with Ca:Na mole ratios ranging from 0.35:1 to 0.45:1. The slurry will comprise from 16 to 26% by weight $Ca(OH)_2$ and from 24 to 34% by weight NaOH. At higher $Ca(OH)_2$ contents, the sodium hydroxide contents will be correspondingly lower, within these ranges of composition.

ii) With stirring to maintain a temperature of about 75°C are added with stirring first the slurry of step i) to a concentrated aqueous solution comprising from 52% to 62% by weight malic acid; second, solid or liquid maleic anhydride is added to the mixture until maleate/malate molar ratios of addition range from 1.1:1 to 1.3:1, calcium to maleate plus malate molar ratios of addition range from 0.4:1 to 0.5:1 and sodium to maleate plus malate molar ratios of addition range from 1.26:1 to 1:46 to 1.

iii) The ether-bond-forming reaction mixture prepared in step ii) is maintained at temperatures of from 65 to 85°C for periods of from 1 to 6 hours, with stirring.

iv) The reaction mixture is then cooled to arrest chemical reaction.

v) The calcium content of the reaction mixture is reduced to a range of from 0.2% to 1% by weight of the 2,2′-oxodisuccinate salts present by treatment with ethane hydroxy diphosphonate, an acidic resin, sodium carbonate, sodium bicarbonate, or combinations thereof.

In either of the above two processes, homogeneous clear liquid reaction mixtures can be obtained.

After the ether-bond forming reaction has been completed to the desired extent, the calcium content of the aqueous reaction mixture may be reduced by conventional means. Removal of calcium can be carried out in a number of ways known in the art. In general, simply adding a calcium precipitating material will suffice. Such calcium precipitating materials include, for example, alkali metal carbonate, ethane hydroxydiphosphonate, pyrophosphate, bicarbonate and/or alkali metal silicate. The resulting calcium precipitate can thereafter be removed from the aqueous reaction product mixture by filtration. In an alternative mode, removing calcium from the aqueous 2,2′-oxodisuccinate reaction product mixture involves treatment of said mixture with an appropriate insoluble ion exchange resin or zeolite. No matter what technique is employed, calcium content of 2,2′-oxodisuccinate salts prepared by methods herein should desirably be reduced to the extent that calcium comprises from 0.2% to 1.0% by weight of the 2,2′-oxodisuccinate in order to form compositions suitable as detergent builders.

2,2′-oxodisuccinate salts formed herein can also be treated, after calcium removal, in a further step, using organic or aqueous solvent extraction to remove excess reactants, such as maleates, or organic reaction by-products, such as fumarates. This can, for example, be accomplished by conventional salt separation procedures using a solvent such as a mixture of methanol and water (4:1v/v) in which these excess reactants and reaction by-products are relatively soluble and in which the desired ether polycarboxylates are relatively insoluble.

At any stage after 2,2′-oxodisuccinate formation, the reaction product can be concentrated by removal of water to the desired extent. Water removal cxan, for example, involve substantially complete drying of the reaction product mixture, e.g., by spray drying, so that the 2,2′-oxodisuccinates are recovered in solid, e.g., granular, form. Alternatively, the sodium 2,2′-oxodisuccinates in the form of an aqueous liquid may be utilized directly in the preparation of detergent compositions or laundry additive products of the types more fully described hereinafter.

After reduction of the calcium content in the reaction product mixture, it is possible, if desired, to acidify the product mixture using conventional acidification or ion exchange techniques to convert the 2,2′-oxodisuccinate salts therein to their free acid form. Normally, however, the 2,2′-oxodisuccinates materials of this invention can, after calcium depletion or complete replacement by sodium, be used as builders in their water-soluble salt form, and such acidification is therefore not usually necessary or desirable.

When converted into suitable form, 2,2′-oxodisuccinates can be used as sequestering builders in a wide variety of detergent compositions or laundry additive compositions. Such builder materials are effective in promoting certain types of fabric cleaning. Such materials retain their efficacy as detergent builders even in relatively low pH cleaning solutions.

Detergent compositions incorporating 2,2′-oxodisuccinates prepared using the ether-bond forming

process improvement of this invention, in detergent-suitable form, contain as essential components from 0.5% to 98% by weight of a surfactant and from 2% to 99.5% by weight of the 2,2'-oxodisuccinate compounds as a detergency builder, generally in sodium-salt form. It is, of course, possible to combine 2,2'-oxodisuccinate detergency builders with known detergency builders in forming such compositions.

More complete disclosures of materials suitable for incorporation in detergent and laundry additive compositions of the present invention are found in US-A 4 056 481, Tate (November 1, 1977); US-A 4 049 586, Collier (September 20, 1977); US-A 4 040 988, Vincent et al (August 9, 1977); US-A 4 035 257, Cherney (July 12, 1977); US-A 4 03 718, Holcolm et al (July 5, 1977); US-A 4 019 999, Ohren et al (April 26, 1977); US-A 4 019 998, Vincent et al (April 26, 1977); and US-A 3 985 669, Krummel et al (October 12, 1976).

In addition to their utility as builders in detergent and laundry additive compositions, the 2,2'-oxodisuccinates of the invention can, after reducing their calcium content, also be utilized in other contexts wherein water hardness sequestration is required. Such contents are provided in water softening compositions, devices and methods and boiler descaling compositions and methods.

The following embodiments illustrate, but are not limiting of, the processes of the present invention. All percentages and parts herein are by weight unlessy indicated otherwise. All ratios herein are mole ratios unless indicated otherwise.

EXAMPLE I

2,2'-OXODISUCCINATE SYNTHESIS (Procedure 1)

DL-malic acid, 134 grams, 1.0 mole, was dissolved in 100 grams of water in a stainless steel reaction vessel equipped with a stirrer and cooling coils. Then, as rapidly as possibly subject to temperature contraints, a slurry of sodium hydroxide, 200 grams, 50% solution, 2.5 moles, and calcium hydroxide, 74 grams, one mole, and 75 grams of water was added while the mixture was stirred and cooled by means of warm water passed through cooling coils in the reaction vessel to maintain a temperature of about 75°C. The reaction was clear and yellowish after about half of the slurry was added, but turned milky after complete addition. Then with care to maintain the reaction temperature at about 75°C, powdered maleic anhydride, 117.6 grams, 1.2 moles was added to the reaction solution. The resulting reaction solution was easily stirred and maintained at 75°C for 12 hours at which time the solution was clear and homogeneous. During the reaction small, 0.1 gram or less samples were removed and analyzed by HPLC. The results are tabulated below. A yield of 68.6% was obtained of 2,2'-oxodisuccinates after 6.0 hours reaction time.

The calcium can be removed from the resulting reaction mixture by treatment with any of the following reagents: ethane hydroxy diphosphonate, an acidic resin or sodium carbonate/sodium bicarbonate.

ANALYSIS OF REACTION MIXTURE WITH TIME (HOURS)

| TIME | MALIC % | MALEIC % | FUMARIC % | ODS % |
|---|---|---|---|---|
| 1.0 | 29.2 | 33.2 | 1.2 | 36.3 |
| 3.0 | 17.9 | 21.1 | 2.4 | 58.6 |
| 6.0 | 13.0 | 14.5 | 4.0 | 68.6 |
| 12.0 | 11.5 | 9.5 | 7.2 | 71.8 |

EXAMPLE II

2,2'-OXODISUCCINATE SYNTHESIS (Procedure 2)

Maleic anhydride 147 g, 1.5 moles, was dissolved in 150 g of water. DL-malic acid, 134 g, 1 mole, was added and this mixture stirred at 70°C until all solids dissolved. With stirring was added a well mixed slurry (consisting of calcium hydroxide 74 g, 1 mole, sodium hydroxide 256 g, 50% solution, 3.2 moles and additional water, 75 g) while the temperature was maintained between 70-85°C. The resulting reaction mixture was stirred and maintained at 75°C for six hours. During the reaction small, 0.1 gram or less, samples were removed and analyzed by HPLC. The results are tabulated below. A yield of 67% was obtained of ODS after six hours.

ANALYSIS OF REACTION MIXTURE WITH TIME (HOURS)

| TIME | MALIC % | MALEIC % | FUMARIC % | ODS % |
|---|---|---|---|---|
| 1.0 | 22.6 | 41.9 | 0.6 | 34.9 |
| 3.0 | 14.7 | 27.1 | 1.9 | 56.3 |
| 6.0 | 10.1 | 17.4 | 5.4 | 67.1 |

## Claims

l. A process for forming sodium and calcium 2,2'-oxodisuccinates characterised in that said process comprises

l. combining, at temperatures below l20°C, in aqueous media to form a reaction mixture an organic reactant component, consisting essentially of

a) at least one maleate reactant selected from

i) acidic or fully neutralised salts, formed from maleate having the empirical formula $C_4H_2O_4$, acidic or neutralising moieties selected from H, sodium and calcium, and combinations thereof,

ii) maleic anhydride,

iii) maleic acid, and

iv) mixtures of said maleate reactants;

and

b) at least one malate reactant, in the form of a single isomer or of isomeric mixtures, selected from

i) acidic or fully neutralised salts, formed from malate having the empirical formula $C_4H_4O_5$, and acidic or neutralising moieties selected from H, sodium and calcium, and combinations thereof,

ii) malic acid, and

iii) mixtures of said malate reactants;

together with an inorganic reactant component, consisting essentially of

a) at least one inorganic base which yields hydroxide anions when placed in water, said base being selected from the oxides and hydroxides of sodium, calcium or mixtures thereof;

or

b) mixtures of said inorganic base with water-soluble, inorganic salts of sodium, calcium or mixtures thereof;

said organic reactant component having a maleate:malate molar ratio ranging from l:l to 2:l; and said inorganic reactant component being used in an amount sufficient to form an alkaline reaction mixture, having initially a molar ratio of calcium to maleate plus malate of from 0.l:l to 0.75:l and a molar ratio of sodium to maleate plus malate of from 0.5:l to 2.2:l;

and

II. maintaining said reaction mixture at a temperature of at least 60°C, sufficient to permit ether-bond formation between said maleate and malate present in the reaction mixture.

2. A process according to Claim l, wherein in forming said reaction mixture, the inorganic reactant component is an inorganic base which is added in an amount which fully neutralises said reaction mixture, and in an amount which is further sufficient to yield an excess of from 0.01 to 0.4 moles of hydroxide per mole of combined maleate and malate.

3. A process according to either of Claim 1 or Claim 2, wherein said organic reactant components are combined in aqueous media to provide a total initial concentration of maleate plus malate, of from 31% to 41% by weight.

4. A process according to any of Claims 1 to 3, wherein the organic and inorganic reactant components are combined using physical agitation, at a temperature of from 60°C to 120°C, and wherein a reaction temperature of from 60°C to 120°C is maintained for a period of from 0.5 hours to 12 hours.

5. A process according to any of Claims 1 to 4, wherein all organic and inorganic reactant components are combined with stirring, at a temperature of from 65°C to 85°C is maintained for a period of from 0.5 hours to 12 hours and wherein chemical reaction is arrested by cooling when at least 50% by weight of the organic species present are in the form of 2,2'-oxodisuccinates.

6. A process according to any of Claims 1 to 5, whereby subsequent to ether-bond formation, 2,2'-oxodisuccinate salts in a form suitable for use as a detergent builder are prepared using conventional calcium removal processes requiring calcium removal agents selected from sodium carbonate, sodium bicarbonate, ethane hydroxy diphosphonate, acidic ion-exchange resins and conventional calcium sequestrants.

7. A process according to any of Claims 1 to 6 wherein said maleate reactants are selected from maleic anhydride, maleic acid and mixtures thereof, said malate reactants are selected from single isomers of malic acid and isomeric mixtures of malic acid; and wherein said inorganic reactant component consists essentially of a mixture of sodium hydroxide and calcium hydroxide.

8. A process according to Claim 7 wherein the reaction mixture is formed using a total initial concentration of maleate plus malate from 31% to 41% by weight of said reaction mixture; and is formed using a total amount of sodium hydroxide which comprises from 5% to 29% by weight of said reaction mixture, calculating said sodium hydroxide as NaOH; and is formed using a total amount of calcium hydroxide which comprises from 2% to 19% by weight of said reaction mixture, calculating said calcium hydroxide as $Ca(OH)_2$.

9. A process for forming 2,2'-oxodisuccinate salts, said process comprising

i) adding dry solid calcium hydroxide to an aqueous sodium hydroxide solution, to form an alkaline slurry comprising from 16% to 20% by weight $Ca(OH)_2$ and from 30 to 34% by weight NaOH; so that the Ca:Na mole ratio therein is from 0.2:1 to 0.4:1;

ii) combining together in water maleic anhydride or maleic acid, and DL-malic acid, using a maleate:malate mole ratio of from 1.4:1 to 1.6:1, at a temperature of from 65°C to 85°C, to form an acidic aqueous solution wherein the concentration of maleate plus malate is from 65% to 75% by weight;

iii) combining the alkaline slurry of step i) and the acidic solution of step ii) with agitation, at a rate selected to avoid precipitation of partially neutralised salts of maleic acid and to form a homogeneous mixture, at a temperature of from 65°C to 85°C, such that the mole ratio of calcium to maleate plus malate is from 0.35:1 to 0.45:1 and the mole ratio of sodium to maleate plus malate is from 1.25:1 to 1.35:1;

iv) mixing and maintaining the reaction mixture which is formed, at a temperature of from 65°C to 85°C, until at least 50% by weight of the organic species present are in the form of 2,2'-oxodisuccinates;

v) cooling said reaction mixture to arrest chemical reaction; and

vi) reducing the calcium content of said reaction mixture, so that calcium comprises from 0.2% to 1% by weight of 2,2'-oxodisuccinate salts present, using ethane hydroxy diphosphonate, an acidic resin, sodium carbonate, sodium bicarbonate, or combinations thereof.

10. A process for forming 2,2'-oxodisuccinate salts, said process comprising

i) preparing an alkaline slurry by combining calcium hydroxide and aqueous sodium hydroxide, wherein Ca:Na mole ratios range from 0.35:1 to 0.45:1, said slurry comprising from 16% to 26% by weight $Ca(OH)_2$ and from 24% to 34% by weight NaOH;

ii) adding successively with stirring to maintain a temperature of about 75°C, first the slurry of step I) to a concentrated aqueous solution comprising from 52% to 62% by weight malic acid; and second, solid or liquid maleic anhydride to the mixture until maleate:malate molar ratios of addition range from 1.1:1 to 1.3:1, calcium to maleate plus malate molar ratios of addition range from 0.4:1 to 0.5:1 and sodium to maleate plus malate molar ratios of addition range from 1.26:1 to 1.46:1;

iii) maintaining the ether-bond forming reaction mixture which is the product of step ii) at temperatures of from 65°C to 85°C for periods of from 1 to 6 hours, with stirring;

iv) cooling said reaction mixture to arrest chemical reaction; and

v) reducing to a range of from 0.2% to 1% by weight of the 2,2'-oxodisuccinate salts present, the calcium content of said reaction mixture, by treatment with ethane hydroxy diphosphonate, an acidic resin, sodium carbonate, sodium bicarbonate, or combinations thereof.

11. A process according to either of Claims 9 or 10 wherein said reaction mixture forms a clear homogeneous liquid.

**Patentansprüche**

1. Verfahren zur Bildung von Natrium- und Calcium-2,2'oxodisuccinaten, dadurch gekennzeichnet, daß das Verfahren die Schritte umfaßt:

I. bei Temperaturen unter 120°C in einem wäßrigen Medium, um eine Reaktionsmischung herzustellen, eine organische Reaktionskomponente, im wesentlichen bestehend aus:

a) wenigstens einem Maleinsäuresalzreaktanden, ausgewählt aus

i) sauren oder vollständig neutralisierten Salzen, gebildet aus Maleat der empirischen Formel $C_4H_2O_4$, und sauren oder neutralisierenden Resten, ausgewählt aus H, Natrium und Calcium und Kombinationen hiervon,

ii) Maleinsäureanhydrid,

iii) Maleinsäure und

iv) Mischungen dieser Maleatreaktanden, und

b) wenigstens einem Malatreaktanden in Form eines einzelnen Isomeren oder Isomerenmischungen, ausgewählt aus

i) sauren oder vollständig neutralisierten Salzen, gebildet aus Malat der empirischen Formel $C_4H_4O_5$ und sauren oder neutralisierenden Resten, ausgewählt aus H, Natrium und Calcium und Kombinationen hiervon,

ii) Äpfelsäure und

iii) Mischungen dieser Malatreaktanden,

mit einer anorganischen Reaktionskomponente mischen, die im wesentlichen besteht aus

a) wenigstens einer anorganischen Base, welche Hydroxidanionen freisetzt, wenn sie in Wasser gegeben wird, und ausgewählt ist aus Oxiden und Hydroxiden von Natrium, Calcium oder Mischungen hiervon, oder

b) Mischungen der anorganischen Base mit wasserlöslichen, anorganischen Salzen von Natrium, Calcium oder deren Mischungen,

wobei die organische Reaktionskomponente ein molares Maleat:Malat-Verhältnis im Bereich von 1:1 bis 2:1 aufweist, und die anorganische Reaktionskomponente in einer Menge eingesetzt wird, die ausreicht, um eine alkalische Reaktionsmischung zu bilden, die anfänglich ein molares Verhältnis von Calcium zu Maleat plus Malat von 0,1:1 bis 0,75:1 und ein molares Verhältnis von Natrium zu Maleat plus Malat von 0,5:1 bis 2,2:1 aufweist, und

II. die Reaktionsmischung bei einer Temperatur von wenigstens 60°C halten, die ausreicht, um die Etherbindungsbildung zwischen dem in der Reaktionsmischung vorhandenen Maleat und dem Malat zu erlauben.

2. Verfahren gemäß Anspruch 1, worin bei der Bildung der Reaktionsmischung die anorganische Reaktionskomponente eine anorganische Base ist, welche in einer Menge zugesetzt wird, die die Reaktionsmischung vollständig neutralisiert und die weiterhin ausreicht, um einen Überschuß von 0,01 bis 0,4 Mol Hydroxid pro Mol Maleat und Malat zusammengenommen zu erzeugen.

3. Verfahren gemäß einem der Ansprüche 1 oder 2, worin die organische und die anorganische Reaktionskomponente in einem wäßrigen Medium zusammengegeben werden, um eine anfängliche Gesamtkonzentration an Maleat plus Malat von 31 bis 41 Gew.-% zu ergeben.

4. Verfahren gemäß einem der Ansprüche 1 bis 3, worin die organische und die anorganische Reaktionskomponente unter Anwendung physikalischen Rührens bei einer Temperatur von 60°C bis 120°C zusammengegeben werden, und worin die Reaktionstemperatur von 60°C bis 120°C für einen Zeitraum von 0,5 bis 12 Stunden aufrechterhalten wird.

5. Verfahren gemäß einem der Ansprüche 1 bis 4, worin alle organischen und anorganischen Reaktionskomponenten unter Rühren bei einer Temperatur von 65°C bis 85°C zusammengegeben werden und worin die Reaktionstemperatur von 65°C bis 85°C für einen Zeitraum von 0,5 bis 12 Stunden aufrechterhalten wird und worin die chemische Reaktion durch Abkühlen abgebrochen wird, wenn wenigstens 50 Gew.-% der vorhandenen organischen Spezies in Form von 2,2'-Oxodisuccinaten vorliegen.

6. Verfahren gemäß einem der Ansprüche 1 bis 5, wobei im Anschluß an die Etherbindungsbildung die 2,2'-Oxodisuccinatsalze in einer für die Verwendung als Detergensgerüststoff geeigneten Form hergestellt werden durch Anwendung konventioneller Verfahren zur Entfernung von Calcium, welche Mittel zur Entfernung von Calcium erforderlich machen, welche ausgewählt sind aus Natriumcarbonat, Natriumbicarbonat, Ethanhydroxydiphosphonat, sauren Ionenaustauscherharzen und konventionellen Calcium-Sequestrierungsmitteln.

7. Verfahren gemäß einem der Ansprüche 1 bis 6, worin die Maleat-Reaktanden ausgewählt sind aus Maleinsäureanhydrid, Maleinsäure und deren Mischungen, und worin die Malat-Reaktanden ausgewählt sind aus einzelnen Isomeren von Äpfelsäure und Isomerenmischungen von Äpfelsäure und worin die anorganische Reaktionskomponente im wesentlichen aus einer Mischung aus Natriumhydroxid und Calciumhydroxid besteht.

8. Verfahren gemäß Anspruch 7, worin die Reaktionsmischung gebildet wird unter Verwendung einer anfänglichen Gesamtkonzentration von Maleat plus Malat von 31 bis 41 Gew.-% der Reaktionsmischung und unter Verwendung einer Gesamtmenge Natriumhydroxid, welche 5 bis 29 Gew.-% der Reaktionsmischung beträgt, wobei Natriumhydroxid als NaOH gerechnet wird, und unter Verwendung einer Gesamtmenge Calciumhydroxid, welche 2 bis 19 Gew.-% der Reaktionsmischung beträgt, wobei Calciumhydroxid als $Ca(OH)_2$ gerechnet wird.

9. Verfahren zur Bildung von 2,2'-Oxodisuccinatsalzen, umfassend:

i) trockenes, festes Calciumhydroxid zu einer wäßrigen Natriumhydroxidlösung geben, um eine alkalische Mischung herzustellen, welche 16 bis 20 Gew.-% $Ca(OH)_2$ und 30 bis 34 Gew.-% NaOH umfaßt, so daß das molare Ca:Na-Verhältnis hierin 0,2:1 bis 0,4:1 beträgt,

ii) Maleinsäureanhydrid oder Maleinsäure und D,L-Äpfelsäure in Wasser bei einer Temperatur von 65°C bis 85°C zusammengeben, wobei ein molares Maleat:Malat-Verhältnis von 1,4:1 bis 1,6:1 angewendet wird, um eine saure wäßrige Lösung zu bilden, worin die Konzentration an Maleat plus Malat 65 bis 75 Gew.-% beträgt,

iii) die alkalische Mischung aus Schritt i) und die saure Lösung aus Schritt ii) unter Rühren bei einer Temperatur von 65 bis 85°C und bei einer Geschwindigkeit zusammengeben, die ausgewählt ist, um das Absetzen von teilweise neutralisierten Salzen von Maleinsäure zu vermeiden und eine homogene Mischung zu bilden, so daß das molare Verhältnis von Calcium zu Maleat plus Malat 0,35:1 bis 0,45:1 und das molare Verhältnis von Natrium zu Maleat plus Malat 1,25:1 bis 1,35:1 beträgt,

iv) die gebildete Reaktionsmischung mischen und bei einer Temperatur von 65°C bis 85°C halten, bis wenigstens 50 Gew.-% der vorhandenen organischen Spezies in Form von 2,2'-Oxodisuccinaten vorliegen,

v) die Reaktionsmischung abkühlen, um die chemische Reaktion abzubrechen, und

vi) den Calcium-Gehalt der Reaktionsmischung verringern, so daß Calcium 0,2 bis 1 Gew.-% der vorhandenen 2,2'-Oxodisuccinatsalze ausmacht, wobei Ethanhydroxydiphosphonat, ein saures Harz, Natriumcarbonat, Natriumbicarbonat oder Mischungen hiervon verwendet werden.

10. Ein Verfahren zur Bildung von 2,2'-Oxodisuccinatsalzen, umfassend die Schritte:

i) eine alkalische Mischung durch Zusammengeben von Calciumhydroxid und wäßrigem Natriumhydroxid herstellen, worin die molaren Ca:Na-Verhältnisse im Bereich zwischen 0,5:1 und 0,45:1 liegen und die Mischung 16 bis 26 Gew.-% $Ca(OH)_2$ und 24 bis 34 Gew.-% NaOH umfaßt,

ii) unter Rühren sukzessiv, um eine Temperatur von etwa 75°C aufrechtzuerhalten, zuerst die Mischung aus Schritt i) einer konzentrierten wäßrigen Lösung, umfassend 52 bis 62 Gew.-% Äpfelsäure, hinzufügen, dann der Mischung festes oder flüssiges Maleinsäureanhydrid hinzugeben, bis die molaren Verhältnisse von hinzugegebenem Maleat:Malat im Bereich zwischen 1,1:1 und 1,3:1 liegen, die molaren Verhältnisse von hinzugegebenem Calcium:Maleat plus Malat im Bereich zwischen 0,4:1 und 0,5:1 liegen, und die molaren Verhältnisse von hinzugegebenem Natrium:Maleat plus Malat im Bereich zwischen 1,26:1 und 1,46:1 liegen,

iii) die Reaktionsmischung der Etherbindungsbildung, welche das Produkt von Schritt ii) ist, bei Temperaturen von 65°C bis 85°C für Zeiträume von 1 bis 6 Stunden unter Rühren aufrechterhalten,

iv) die Reaktionsmischung abkühlen, um die chemische Reaktion abzubrechen,

v) den Calcium-Gehalt der Reaktionsmischung auf einen Bereich von 0,2 bis 1 Gew.-% der vorhandenen 2,2'-Oxodisuccinatsalze verringern, durch Behandlung mit Ethanhydroxydiphosphonat, einem sauren Harz, Natriumcarbonat, Natriumbicarbonat oder Mischungen hiervon.

11. Verfahren gemäß einem der Ansprüche 9 oder 10, worin die Reaktionsmischung eine klare homogene Flüssigkeit bildet.

**Revendications**

1. Procédé de formation de 2,2'-oxodisuccinates de sodium et de calcium, caractérisé en ce que ledit procédé comprend

I. La combinaison, à des températures inférieures à 120°C, en milieu aqueux pour former un mélange réactionnel, d'un composant réactif organique, constitué essentiellement de

a) au moins un réactif de maléate choisi parmi

i) les sels acides ou pleinement neutralisés, formés à partir de maléate ayant la formule brute $C_4H_2O_4$, des fractions acides ou neutralisantes choisies parmi H, le sodium et le calcium et leurs combinaisons,

ii) l'anhydride maléique,

iii) l'acide maléique, et

iv) les mélanges desdits réactifs de maléate;

et

b) au moins un réactif de malate, sous la forme d'un seul isomère, ou de mélanges isomériques, choisi parmi

i) les sels acides ou pleinement neutralisés, formés à partir de malate, ayant la formule brute $C_4H_4O_5$ et les fractions acides ou neutralisantes choisies parmi H, le sodium et le calcium, ou leurs combinaisons,

ii) l'acide malique, et

iii) les mélanges desdits réactifs de malate;

avec un composant réactif minéral, constitué essentiellement de

a) au moins une base minérale, qui donne des anions hydroxyde lorsqu'on la met dans l'eau, ladite base étant choisie parmi les oxydes et hydroxydes de sodium, calcium, et leurs mélanges;

ou

b) les mélanges de ladite base minérale avec des sels minéraux solubles dans l'eau de sodium, de calcium ou leurs mélanges;

ledit composant réactif organique ayant un rapport molaire maléate:malate allant de 1:1 à 2:1; et ledit composant réactif minéral étant utilisé en une quantité suffisante pour former un mélange réactionnel alcalin, ayant essentiellement un rapport molaire du calcium au maléate + malate allant de 0,1:1 à 0,75:1 et un rapport molaire du sodium au maléate + malate allant de 0,5:1 à 2,2:1;

et

II. Le maintien dudit mélange réactionnel à une température d'au moins 60°C, suffisante pour permettre la formation d'une liaison éther entre lesdits maléate et malate présents dans le mélange réactionnel.

2. Procédé selon la revendication 1, dans lequel dans la formation dudit mélange réactionnel, le composant réactif minéral est une base minérale qui est ajoutée en une quantité qui neutralise pleinement ledit mélange réactionnel et en une quantité qui est en outre suffisante pour donner un excès de 0,01 à 0,4 mole d'hydroxyde par mole de maléate et malate combinés.

3. Procédé selon la revendication 1 ou la revendication 2, dans lequel lesdits composants réactifs organique et minéral sont combinés en milieu aqueux pour fournir une concentration initiale totale de maléate + malate allant de 31 à 41% en poids.

4. Procédé selon l'une quelconque des revendications 1 à 3, dans lequel les composants réactifs organique et minéral sont combinés en utilisant une agitation physique, à une température allant de 60°C à 120°C et où on maintient une température réactionnelle allant de 60°C à 120°C pendant une période allant d'½ heure à 12 heures.

5. Procédé selon l'une quelconque des revendications 1 à 4, dans lequel tous les composants réactifs

organiques et minéraux sont combinés en agitant à une température allant de 65°C à 85°C, et où une température réactionnelle allant de 65°C à 85°C est maintenue pendant une période allant de ½ heure à 12 heures, et où la réaction chimique est arrêtée par refroidissement lorsqu'au moins 50% en poids de l'espèce organique présente sont sous forme de 2,2'-oxodisuccinates.

6. Procédé selon l'une quelconque des revendications 1 à 5, dans lequel après la formation de la liaison éther, on prépare les sels de 2,2'-oxodisuccinate sous une forme appropriée pour utilisation comme auxiliaire de détergence en utilisant des procédés classiques d'enlèvement du calcium nécessitant des agents d'enlèvement du calcium choisis parmi le carbonate de sodium, le bicarbonate de sodium, l'éthanehydroxy-diphosphonate, les résines échangeuses d'ions acides et les séquestrants de calcium classiques.

7. Procédé selon l'une quelconque des revendications 1 à 6, dans lequel lesdits réactifs de maléate sont choisis parmi l'anhydride maléique, l'acide maléique et leurs mélanges, lesdits réactifs de maléate sont choisis parmi les isomères isolés d'acide malique et les mélanges isomériques d'acide malique;
et où ledit composant réactif minéral consiste essentiellement en un mélange d'hydroxyde de sodium et d'hydroxyde de calcium.

8. Procédé selon la revendication 7, dans lequel le mélange réactionnel est formé en utilisant une concentration initiale totale de maléate + malate allant de 31% à 41% en poids dudit mélange réactionnel;
et est formé en utilisant une quantité totale d'hydroxyde de sodium qui comprend de 5% à 29% en poids dudit mélange réactionnel, en calculant ledit hydroxyde de sodium sous forme de NaOH, et est formé en utilisant une quantité totale d'hydroxyde de calcium qui comprend de 2% à 19% en poids dudit mélange réactionnel, en calculant ledit hydroxyde de calcium sous forme de $Ca(OH)_2$.

9. Procédé de formation de sels de 2,2'-oxodisuccinate, lequel procédé comprend
i) l'addition d'hydroxyde de calcium solide sec à une solution aqueuse d'hydroxyde de calcium, pour former une bouillie alcaline comprenant de 16% à 20% en poids de $Ca(OH)_2$ et de 30 à 34% en poids de NaOH; pour que le présent rapport molaire Ca:Na soit de 0,2:1 à 0,4:1;
ii) la combinaison ensemble dans l'eau, d'anhydride maléique ou d'acide maléique, et d'acide DL-malique, en utilisant un rapport molaire maléate:malate allant de 1,4:1 à 1,6:1, à une température allant de 65°C à 85°C, pour former une solution aqueuse acide dans laquelle la concentration de maléate + malate est de 65% à 75% en poids;
iii) la combinaison de la bouillie alcaline de l'étape i) et de la solution acide de l'étape ii) avec agitation, à une vitesse choisie pour éviter la précipitation des sels partiellement neutralisés de l'acide maléique et pour former un mélange homogène, à une température allant de 65°C à 85°C, de manière que le rapport molaire du calcium au maléate + malate soit de 0,35:1 à 0,45:1 et que le rapport molaire du sodium au maléate + malate soit de 1,25:1 à 1,35:1;
iv) le mélange et le maintien du mélange réactionnel qui est formé à une température allant de 65°C à 85°C, jusqu'à ce qu'au moins 50% en poids des espèces organiques présentes soient sous forme de 2,2'-oxodisuccinates ;
v) le refroidissement dudit mélange réactionnel pour arrêter la réaction chimique;
vi) la réduction de la teneur en calcium dudit mélange réactionnel, pour que le calcium constitue de 0,2% à 1% en poids des sels de 2,2'-oxodisuccinate présents, en utilisant de l'éthane-hydroxy-diphosphonate, une résine acide, du carbonate de sodium, du bicarbonate de sodium, ou leurs combinaisons.

10. Procédé de formation de sels de 2,2'-oxodisuccinate, ledit procédé comprenant
i) la préparation d'une bouillie alcaline par combinaison d'hydroxyde de calcium et d'hydroxyde de sodium aqueux, où les rapports molaires Ca:Na vont de 0,35:1 à 0,45:1, ladite bouillie comprenant de 16% à 26% en poids de $Ca(OH)_2$ et de 24% à 34% en poids de NaOH;
ii) l'addition successivement avec agitation pour maintenir une température d'environ 75°C, tout d'abord de la bouillie de l'étape i) à une solution aqueuse concentrée comprenant de 52% à 62% en poids d'acide malique, et en second lieu d'anhydride maléique solide ou liquide au mélange jusqu'à ce que les rapports molaires d'addition maléate:malate aillent de 1,1:1 à 1,3:1, les rapports molaires d'addition du calcium au maléate + malate aillent de 0,4:1 à 0,5:1 et les rapports molaires d'addition du sodium au maléate + malate aillent de 1,26:1 à 1,46:1;
iii) le maintien du mélange réactionnel formateur de liaison éther qui est le produit de l'étape ii) à des températures allant de 65°C à 85°C pendant des durées allant de 1 à 6 heures, tout en agitant;
iv) le refroidissement dudit mélange réactionnel pour arrêter la réaction chimique;
v) la réduction à un intervalle allant de 0,2% à 1% en poids des sels de 2,2'-oxodisuccinate présents, de la teneur en calcium dudit mélange réactionnel, par traitement avec de l'éthane-hydroxydiphosphonate, une résine acide, du carbonate de sodium, du bicarbonate de sodium, ou leurs combinaisons.

11. Procédé selon les revendications 9 ou 10, dans lequel ledit mélange réactionnel forme un liquide homogène clair.